(19) Europäisches Patentamt
European Patent Office
Office européen des brevets

(11) **EP 4 335 836 A1**

(12) **EUROPÄISCHE PATENTANMELDUNG**

(43) Veröffentlichungstag:
   **13.03.2024   Patentblatt 2024/11**

(21) Anmeldenummer: **22195156.9**

(22) Anmeldetag: **12.09.2022**

(51) Internationale Patentklassifikation (IPC):
   *C07C 1/20* (2006.01)     *C07C 11/04* (2006.01)
   *C07C 11/06* (2006.01)    *C07C 11/08* (2006.01)
   *C07C 41/09* (2006.01)    *C07C 43/04* (2006.01)
   *C07C 4/06* (2006.01)

(52) Gemeinsame Patentklassifikation (CPC):
   (C-Sets verfügbar)
   **C07C 1/20; C07C 4/06; C07C 41/09**     (Forts.)

(84) Benannte Vertragsstaaten:
   **AL AT BE BG CH CY CZ DE DK EE ES FI FR GB
   GR HR HU IE IS IT LI LT LU LV MC MK MT NL NO
   PL PT RO RS SE SI SK SM TR**
   Benannte Erstreckungsstaaten:
   **BA ME**
   Benannte Validierungsstaaten:
   **KH MA MD TN**

(71) Anmelder: **BASF SE**
   **67056 Ludwigshafen am Rhein (DE)**

(72) Erfinder:
   • **BADER, Andre**
     **67063 Ludwigshafen am Rhein (DE)**

   • **COLLINS, Lee Russell**
     **67056 Ludwigshafen am Rhein (DE)**
   • **JOERKE, Andreas**
     **67056 Ludwigshafen am Rhein (DE)**
   • **STROBEL, Paul-Vinzent**
     **67056 Ludwigshafen am Rhein (DE)**
   • **FRANZ, Robert Peter Michael**
     **67056 Ludwigshafen am Rhein (DE)**

(74) Vertreter: **Schuck, Alexander**
   **Patentanwälte**
   **Isenbruck Bösl Hörschler PartG mbB**
   **Eastsite One**
   **Seckenheimer Landstraße 4**
   **68163 Mannheim (DE)**

(54) **VERFAHREN ZUR HERSTELLUNG VON C2-C4-OLEFINEN AUS METHANOL UND ETHANOL**

(57)     Die Erfindung betrifft ein Verfahren zur Herstellung von $C_2$-$C_4$-Olefinen aus Methanol und Ethanol mit den Schritten:

A) Einspeisen eines Methanol und gegebenenfalls Ethanol enthaltenden Einspeisungsstroms A in einen Dimethylether-Festbettreaktor und katalytische Umsetzung von Methanol zu Dimethylether, wobei ein Produktstrom A1 enthaltend Dimethylether, Methanol, Ethanol und Wasserdampf erhalten wird;

B) Mischen des Stroms A1 mit mindestens einem Kohlenwasserstoff-Rückführstrom R enthaltend $C_2$-$C_6$-Kohlenwasserstoffe und katalytische Umsetzung in einem Olefin-Festbettreaktor zu einem Rohproduktstrom B enthaltend $C_2$-$C_4$-Olefine, $C_5$-$C_6$-Kohlenwasserstoff und $C_7^+$-Kohlenwasserstoffe;

C) Abkühlung des Rohproduktstroms B, wobei ein Kohlenwasserstoff-Rohproduktstrom C erhalten wird;

D) Auftrennung des Kohlenwasserstoff-Rohproduktstroms C in einen Propylen enthaltenden Wertproduktstrom, gegebenenfalls einen Ethylen enthaltenden Wertproduktstrom, einen Butene enthaltenden Wertproduktstrom, mindestens einen $C_5$-$C_6$-Kohlenwasserstoff enthaltenden Rückführstrom und mindestens einen $C_6^+$-Kohlenwasserstoffe enthaltenden Nebenproduktstrom;

E) Rückführung eines Teils der $C_2$-$C_4$-Oefine und mindestens eines Teils der $C_5$-$C_6$-Kohlenwasserstoffe als ein oder mehrere Kohlenwasserstoff-Rückführströme in Schritt B);

F) Gewinnung eines Propylen enthaltenden Wertproduktstroms, eines Ethylen enthaltenden Wertproduktstroms und gegebenenfalls eines Butene enthaltenen Wertproduktstroms;

G) Ausschleusung des $C_6^+$-Kohlenwasserstoffe enthaltenden Nebenproduktstroms; dadurch gekennzeichnet, dass

der Strom A, bezogen auf Methanol und Ethanol, < 1 Gew.-% oder 30 bis 50 Gew.-% Ethanol enthält, wobei, bezogen auf 100 Gew.-% der als Wertprodukte gewonnenen C2-C4-Olefine, 30 bis 60 Gew.-% Ethylen, 30 bis 60 Gew.-% Propylen und 0 bis 30 Gew.-% Butene als Wertprodukte gewonnen werden, und, bezogen auf die in dem Rohproduktstrom B enthaltenen $C_2$-$C_4$-Olefine, 0 bis 40% des Ethylens, 40 bis 90% des Propylens und 0 bis 100% der Butene in Schritt B) zurückgeführt werden,

oder der Strom A, bezogen auf Methanol und Ethanol, 1 bis 30 Gew.-% Ethanol enthält, wobei, bezogen auf 100 Gew.-% der als Wertprodukte gewonnenen $C_2$-$C_4$-Olefine, 0 bis 20 Gew.-% Ethylen, 70 bis 100 Gew.-% Propylen und 1 bis 20 Gew.-% Butene als Wertprodukte gewonnen werden, und, bezogen auf die in dem Rohpro-

EP 4 335 836 A1

duktstrom B enthaltenen C2-C4-Oefine, 0 bis 100 % des Ethylens, 0 bis 20 % des Propylens und 40 bis 100% der Butene in Schritt B) zurückgeführt werden.

(52) Gemeinsame Patentklassifikation (CPC): (Forts.)

C-Sets
**C07C 1/20, C07C 11/04;**
**C07C 1/20, C07C 11/06;**
**C07C 1/20, C07C 11/08;**
**C07C 4/06, C07C 11/04;**
**C07C 4/06, C07C 11/06;**
**C07C 4/06, C07C 11/08;**
**C07C 41/09, C07C 43/043**

**Beschreibung**

Die Erfindung betrifft ein Verfahren zur Herstellung von $C_2$-$C_4$-Olefinen aus Methanol und gegebenenfalls Ethanol.

Es ist bekannt, Propylen durch Umsetzung eines Methanol/Dimethylether-Gemischs in einem Festbettreaktor (Methanol-to-Propylene, MTP-Reaktor) zu erzeugen. Bekannte Festbettreaktoren werden mit Zeolith-Katalysatoren bei Temperaturen von ca. 480°C betrieben.

US 2010/145125 A1 offenbart ein Verfahren zur Herstellung leichter Olefine durch die Umwandlung von Methanol und Ethanol. Das Verfahren umfasst das Zuführen eines ersten Teilfeeds über einen Verteiler am Boden eines Wirbelschichtreaktors zu einer Reaktionszone, die einen Katalysator enthält, das Zuführen eines zweiten Teilfeeds von mindestens einer Stelle oberhalb des Verteilers zu der Reaktionszone, das Kontaktieren des Feeds mit dem Katalysator und Reagieren lassen zu einem Strom enthaltend Ethylen und Propylen, wobei der erste und der zweite Teilfeed jeweils unabhängig voneinander Methanol und/oder Ethanol umfassen mit der Maßgabe, dass der Feed insgesamt sowohl Methanol als auch Ethanol umfasst und das Gewichtsverhältnis von Methanol zu Ethanol in dem Feed insgesamt im Bereich von 99:1 bis 0,1:1 liegt.

CN 216106699 U offenbart ein Verfahren zur Herstellung von Ethylen, Propylen und Butenen durch katalytische Dehydratisierung von Methanol. Das System umfasst eine Reaktionseinheit und eine Trenneinheit, wobei die Reaktionseinheit einen Vorreaktor, eine Prozessdampfsäule und einen Hauptreaktor und die Trenneinheit einen Quencher und einen Kompressor umfasst. Der Hauptreaktor enthält einen ZSM-5-Molekularsiebkatalysator, die Trenneinheit umfasst eine Ethylen-Kolonne, eine Propylen-Kolonne und eine Butene-Kolonne. Ethan, Propan, Butan und $C_5$- und $C_6$-Kohlenwasserstoffen werden teilweise in den Hauptreaktor zurückgeführt, weitere Komponenten werden als Nebenprodukte ausgeschleust. Mit der Einheit können auch Butene hergestellt werden, während die Menge an rückgeführten Kohlenwasserstoffen verringert und der Energieverbrauch der Einheit gesenkt wird. Es werden nur gesättigte Kohlenwasserstoffe in den Hauptreaktor zurückgeführt.

CN 110218138 A offenbart ein Verfahren zur Erhöhung der Ausbeute von Olefin in einem Methanol-zu-Propylen-Verfahren (MTP-Verfahren), bei dem $C_2$-Kohlenwasserstoffe, $C_4$-Kohlenwasserstoffe und $C_5$-$C_7$-Kohlenwasserstoffe in den MTP-Reaktor zurückgeführt werden. In einem Beispiel beträgt in einer 500 000 Jahrestonnen Methanol-zu-Propylen-Anlage die Methanolzufuhr 210 t/h und die der zurückgeführten $C_2$-, $C_4$- und $C_5$-$C_7$-Kohlenwasserstoffe 27 t/h, 40 t/h bzw. 150 t/h. Die Ethylenproduktion beträgt 1,5 t/h, die Propylenproduktion beträgt 60 t/h. Die Co-Einspeisung von Ethanol wird nicht erwähnt. Es wird ganz überwiegend Propylen als Wertprodukt erhalten.

Aufgabe der Erfindung ist es, ein flexibles Verfahren zur Herstellung von $C_2$-$C_4$-Olefinen aus Methanol in einem Methanol-To-Olefin-Prozess (MTO-Prozess) bereitzustellen, bei dem die Anteile an Ethylen, Propylen und Butenen in den aus dem Verfahren gewonnenen Wertproduktströmen in weiten Bereichen variiert werden können und die Menge der in den MTO-Prozess rezyklierten Rückführströme insgesamt verringert werden kann.

Gelöst wird die Aufgabe durch ein Verfahren zur Herstellung von $C_2$-$C_4$-Olefinen aus Methanol und gegebenenfalls Ethanol mit den Schritten:

A) Einspeisen eines Methanol und gegebenenfalls Ethanol enthaltenden Einspeisungsstroms A in einen Dimethylether-Festbettreaktor und katalytische Umsetzung von Methanol zu Dimethylether, wobei ein Produktstrom A1 enthaltend Dimethylether, Methanol, Wasserdampf und gegebenenfalls Ethanol und Ethylen erhalten wird;

B) Mischen des Stroms A1 mit mindestens einem Kohlenwasserstoff-Rückführstrom R enthaltend $C_2$-$C_6$-Kohlenwasserstoffe und katalytische Umsetzung in einem Olefin-Festbettreaktor zu einem Rohproduktstrom B enthaltend $C_2$-$C_4$-Olefine, $C_5$-$C_6$-Kohlenwasserstoff und $C_7^+$-Kohlenwasserstoffe;

C) Abkühlung des Rohproduktstroms B, wobei ein Kohlenwasserstoff-Rohproduktstrom C erhalten wird;

D) Auftrennung des Kohlenwasserstoff-Rohproduktstroms C in einen Propylen enthaltenden Wertproduktstrom, gegebenenfalls einen Ethylen enthaltenden Wertproduktstrom, einen Butene enthaltenden Wertproduktstrom, in mindestens einen $C_5$-$C_6$-Kohlenwasserstoff enthaltenden Rückführstrom und in mindestens einen $C_6^+$-Kohlenwasserstoffe enthaltenden Nebenproduktstrom;

E) Rückführung eines Teils der $C_2$-$C_4$-Oefine und mindestens eines Teils der $C_5$-$C_6$-Kohlenwasserstoffe als ein oder mehrere Kohlenwasserstoff-Rückführströme R in Schritt B);

F) Gewinnung eines Propylen enthaltenden Wertproduktstroms, eines Ethylen enthaltenden Wertproduktstroms und gegebenenfalls eines Butene enthaltenden Wertproduktstroms;

G) Ausschleusung des $C_6^+$-Kohlenwasserstoffe enthaltenden Nebenproduktstroms;

dadurch gekennzeichnet, dass der Strom A, bezogen auf Methanol und Ethanol, < 1 Gew.-% oder 30 bis 50 Gew.-% Ethanol enthält, wobei, bezogen auf 100 Gew.-% der als Wertprodukte gewonnenen $C_2$-$C_4$-Olefine, 30 bis 60 Gew.-% Ethylen, 30 bis 60 Gew.-% Propylen und 0 bis 30 Gew.-% Butene als Wertprodukte gewonnen werden, und, bezogen auf die in dem Rohproduktstrom B enthaltenen $C_2$-$C_4$-Oefine, 0 bis 40% des Ethylens, 40 bis 90% des Propylens und 0 bis 100% der Butene in Schritt B) zurückgeführt werden,

oder der Strom A, bezogen auf Methanol und Ethanol, 1 und 30 Gew.-% Ethanol enthält, wobei, bezogen auf 100 Gew.-% der als Wertprodukte gewonnenen $C_2$-$C_4$-Olefine, 0 bis 20 Gew.-% Ethylen, 70 bis 100 Gew.-% Propylen und 1 bis 20 Gew.-% Butene als Wertprodukte gewonnen werden, und, bezogen auf die in dem Rohproduktstrom B enthaltenen $C_2$-$C_4$-Oefine, 0 bis 100 % des Ethylens, 0 bis 20 % des Propylens und 40 bis 100% der Butene in Schritt B) zurückgeführt werden.

In dem erfindungsgemäßen Verfahren können, bezogen auf 100 Gew.-% der als Wertprodukte gewonnenen $C_2$-$C_4$-Olefine, 30 bis 60 Gew.-% Ethylen, 30 bis 60 Gew.-% Propylen und 0 bis 30 Gew.-% Butene als Wertprodukte gewonnen werden, und, bezogen auf die in dem Rohproduktstrom B enthaltenen $C_2$-$C_4$-Oefine, 0 bis 40% des Ethylens, 40 bis 90% des Propylens und 0 bis 100% der Butene in Schritt B) zurückgeführt werden. Der Anteil des Ethanols an den insgesamt dem Prozess zugeführten Alkoholen Ethanol und Methanol kann bei < 1 Gew.-%, insbesondere 0 Gew.-% liegen. Alternativ kann zur Verbesserung der Prozessökonomie der Anteil des Ethanols an den dem Prozess zugeführten Alkoholen bei 30 bis 50 Gew.-% liegen.

Alternativ kann der Anteil des Ethanols an den insgesamt dem Prozess zugeführten Alkoholen auch zwischen 1 und 30 Gew.-% liegen wobei, bezogen auf 100 Gew.-% der als Wertprodukte gewonnenen $C_2$-$C_4$-Olefine, 0 bis 20 Gew.-% Ethylen, 70 bis 100 Gew.-% Propylen und 1 bis 20 Gew.-% Butene als Wertprodukte gewonnen werden, und, bezogen auf die in dem Rohproduktstrom B enthaltenen $C_2$-$C_4$-Oefine, 0 bis 100 % des Ethylens, 0 bis 20 % des Propylens und 40 bis 100% der Butene in Schritt B) zurückgeführt werden.

Es wurde gefunden, dass durch die veränderte Rückführungsvarianten Produktzusammensetzungen erreicht werden können, die ansonsten nur durch sehr aufwendige Katalysatoroptimierungen möglich wären. Außerdem sorgt eine Zugabe von Ethanol in den ermittelten Vorzugsbereichen für eine deutlich reduzierte Menge an Rückführstrom und Nebenprodukten. Dies reduziert den Energiebedarf des Prozesses signifikant, da z.B. weniger Masse nach dem Reaktor aufwendig aufgetrennt werden muss.

In einer bevorzugten Ausführungsform umfasst das erfindungsgemäße Verfahren die Schritte:

A1) Einspeisen eines Methanol und gegebenenfalls Ethanol enthaltenden Einspeisungsstroms A in einen Dimethylether-Festbettreaktor und katalytische Umsetzung von Methanol zu Dimethylether und gegebenenfalls Ethanol zu Ethylen, wobei ein Produktstrom A1 enthaltend Dimethylether, Methanol, Wasserdampf und gegebenenfalls Ethanol und Ethylen erhalten wird;

A2) Mischen von mindestens einem Teil des Produktstroms A1 mit mindestens einem Kohlenwasserstoff-Rückführstrom R enthaltend $C_2$-$C_6$-Kohlenwasserstoffe und einem Wasserdampfstrom, wobei ein zweiter Einspeisungsstrom A2 erhalten wird;

B1) Aufheizen des zweiten Einspeisungsstroms A2 in einem oder mehreren Wärmetauschern auf eine Temperatur im Bereich von 430 bis 500°C und Einspeisung in einen Olefin-Festbettreaktor, wobei das Aufheizen auch vor dem Mischen einzelner Teilströme zum Einspeisungsstrom A2 in Schritt A2) stattfinden kann;

B2) katalytische Umsetzung des Einspeisungsstroms A2 bei einer Temperatur im Bereich von 430 bis 520°C zu einem Rohproduktgasstrom B enthaltend Ethylen, Propylen, Butene, weitere $C_2$-$C_6$-Kohlenwasserstoffe, $C_7^+$-Kohlenwasserstoffe, Methanol und Wasserdampf;

C1) Abkühlen des Rohproduktgasstroms B in einem oder mehreren Wärmetauschern auf eine Temperatur im Bereich von 170 bis 220°C durch Wärmetausch mit dem Einspeisungsstrom A2;

C2) weitere Abkühlung des Rohproduktgasstroms B auf eine Temperatur im Bereich von 30 bis 60°C durch Inkontaktbringen mit mindestens einem wasserhaltigem Quench-Kreislaufstrom K, wobei Wasser und Methanol auskondensiert werden und ein an Wasser und Methanol abgereicherter Kohlenwasserstoff-Rohproduktgasstrom C erhalten wird;

D) Auftrennung des Kohlenwasserstoff-Rohproduktgasstrom C in einen Propylen enthaltenden Wertproduktstrom, gegebenenfalls einen Ethylen enthaltenden Wertproduktstrom, einen Butene enthaltenden Wertproduktstrom, min-

destens einen $C_5$-$C_6$-Kohlenwasserstoffe enthaltenden Rückführstrom und mindestens einen $C_6^+$-Kohlenwasserstoffe enthaltenden Nebenproduktstrom.

In Schritt A1) wird ein Methanol und Ethanol enthaltender Einspeisungsstrom A in einen Dimethylether-Festbettreaktor eingespeist und Methanol zu Dimethylether katalytisch umgesetzt, wobei ein Produktstrom A1 enthaltend Dimethylether, Methanol, Ethanol und Wasserdampf erhalten wird.

Wenn, bezogen auf 100 Gew.-% der als Wertprodukte gewonnenen $C_2$-$C_4$-Olefine, 30 bis 60 Gew.-% Ethylen, 30 bis 60 Gew.-% Propylen und 0 bis 30 Gew.-% Butene als Wertprodukte gewonnen werden, kann der Anteil des Ethanols an den insgesamt dem Prozess zugeführten Alkoholen Ethanol und Methanol bei <1 Gew.-% liegen. Alternativ kann der Anteil an Ethanol auf 30 bis 50 Gew.-% erhöht werden, um ein günstigeres Betriebsfenster zu erreichen.

Alternativ liegt ein vorteilhafter Anteil des Ethanols an den insgesamt dem Prozess zugeführten Alkoholen Ethanol und Methanol bei 1 bis 30 Gew.-%, wenn, bezogen auf 100 Gew.-% der als Wertprodukte gewonnenen $C_2$-$C_4$-Olefine, 1 bis 20 Gew.-% Ethylen, 70 bis 100 Gew.-% Propylen und 0 bis 20 Gew.-% Butene als Wertprodukte gewonnen werden.

Der Dimethyletherfestbettreaktor kann auf verschiedene Arten konzeptioniert sein, je nachdem wie der Einspeisungsgasstrom genau zusammengesetzt ist. Im Allgemeinen wird der Methanol und gegebenenfalls Ethanol enthaltende Einspeisungsgasstrom A aufgeheizt und auf eine Temperatur von über 250 °C erhitzt und in den Festbettreaktor eingespeist. Als Katalysator dient im Allgemeinen gamma-Aluminiumoxid. Die Temperatur der Umsetzung liegt zwischen 250 und 450 °C und der Druck zwischen 1 und 25 bar, beispielsweise 4 bar. Der Methanol-Umsatz beträgt im Allgemeinen 50 bis 90%, vorzugsweise 65 bis 85%, beispielsweise 75%.

Bei geringen Ethanol-Anteilen kann der Strom A auf eine Temperatur von 250 bis 300 °C, beispielsweise 275 °C, vorgeheizt werden. Der den Reaktor verlassende Produktgasstrom A1 weist dann eine Temperatur von im Allgemeinen 350 bis 400 °C, z.B. 370 °C auf. Dieser Strom wird dann durch Wärmetausch mit Strom A auf eine Temperatur von im Allgemeinen 180 bis 250 °C abgekühlt.

Bei höheren Ethanolanteilen in Strom A kann Strom A auch stattdessen auf Temperaturen von bis zu 450 °C aufgeheizt und der Reaktor adiabat betrieben werden. Alternativ kann der Reaktor als beheizter Reaktor konzipiert werden, wobei Strom A dann lediglich auf Temperaturen von bis zu 400 °C aufgeheizt werden muss.

In einem Schritt A2) wird zumindest ein Teil dieses Stroms A1 mit einem Kohlenwasserstoff-Rückführstrom R enthaltend $C_2$-$C_6$-Kohlenwasserstoffe und einem Wasserdampfstrom gemischt, wobei ein Einspeisungsgasstrom A2 erhalten wird. Im Allgemeinen beträgt dieser Teil des Stroms A1 mindestens 50 Gew.-% und vorzugsweise bis zu 90 Gew.-%. Ein weiterer Teil A1-2 des Stroms A1 von vorzugsweise mindestens 10 Gew.-% kann direkt einer oder mehreren Horden des Olefin-Festbettreaktors zugeführt werden. Dieser Teil des Stroms A1 wird im Allgemeinen vor der Einspeisung in die Horden des Olefin-Festbettreaktors gekühlt, vorzugsweise auf eine Temperatur im Bereich von 30 bis 60°C. Dieser Teilstrom A1-2 wird vorzugsweise flüssig in den Reaktor eingespeist.

Die Kühlung durch den Teilstrom A1-2 kann auch entfallen, wenn der Olefin-Festbettreaktor isotherm betrieben und gekühlt wird. Ein isothermer Betrieb kann beispielsweise in der in WO 2017/102096 A1 beschrieben Art und Weise erfolgen. So können in den Olefin-Festbettreaktor Wärmeübertragerflächen eingebaut sein, die beispielweise mit Flüssigsalz oder Hochdruckdampf als Wärmeübertragermedium betrieben werden. Indem das Wärmeübertragermedium durch die Wärmeübertragerflächen in dem Reaktor strömt, wird die entstehende Reaktionswärme aus dem Reaktor abgeführt und dieser somit isotherm betrieben. Zur Einstellung der gewünschten Produktverteilung in dem Produktgasstrom B kann auch hier ein Teilstrom A1-2 einer Zwischenstufe des Olefin-Festbettreaktors zugeführt werden.

Der mindestens eine Kohlenwasserstoff-Rückführstrom R, der aus der Abtrennung der $C_2$-$C_4$-Olefine stammt, enthält im Allgemeinen $C_1$-$C_6$-Kohlenwasserstoffe. Je nachdem, in welchen Anteilen Ethylen, Propylen und Butene in Schritt E) als Wertprodukte gewonnen werden, enthält der mindestens eine Kohlenwasserstoff-Rückführstrom $C_2$-$C_4$ Kohlenwasserstoffe, im Allgemeinen in Mengen von insgesamt 50. bis 90. Gew.-%. Der mindestens eine Kohlenwasserstoff-Rückführstrom weist vor dem Vermischen im Allgemeinen eine Temperatur im Bereich von 100 bis 175°C, bevorzugt im Bereich von 130 bis 160°C auf.

Der Produktstrom A1 des Dimethylether-Festbettreaktors wird weiterhin mit einem Wasserdampfstrom gemischt. Der Wasserdampfstrom weist im Allgemeinen eine Temperatur im Bereich von 100 bis 200°C, bevorzugt im Bereich von 100 bis 150°C auf. Der so erhaltene Einspeisungsstrom A2 enthält im Allgemeinen 20 bis 70 Gew.-%, vorzugsweise 30 bis 60 Gew.-% Wasserdampf. Er enthält im Allgemeinen weiterhin 5 bis 10 Gew.-% Methanol, 0 bis 15 Gew.-% Ethanol, 10 bis 20 Gew.-% Dimethylether und 15 bis 50 Gew.-% $C_2$-$C_6$-Kohlenwasserstoffe.

In einem Schritt B1) wird der Einspeisungsstrom A2 in einem oder mehreren Wärmetauschern auf eine Temperatur im Bereich von im Allgemeinen 430 bis 500°C aufgeheizt und in einen Olefin-Festbettreaktor eingespeist. Das Aufheizen kann auch vor dem Mischen einzelner Teilströme in Schritt A2) stattfinden.

Im Allgemeinen weist der Einspeisungsstrom A2 beim Einspeisen in den Olefin-Festbettreaktor eine Temperatur im Bereich von 430 bis 500°C, beispielsweise von 470°C auf. Das Aufheizen des Einspeisungsstroms A2 auf diese Temperatur kann

durch Wärmetausch mit dem Rohproduktgasstrom B des Olefin-Festbettreaktors, einer direkten elektrischen Beheizung oder Beheizung durch Verbrennung eines separaten fossilen Energieträgers erfolgen.

Es folgt in Schritt B2) die katalytische Umsetzung in dem Olefin-Festbettreaktor zu einem Produktgasstrom B enthaltend Ethylen, Propylen, Butene, weitere $C_2$-$C_6$-Kohlenwasserstoffe, $C_7^+$-Kohlenwasserstoffe, Methanol und Wasserdampf. Die Umsetzung erfolgt im Allgemeinen an einem Zeolith-Katalysator, bevorzugt an einem Katalysator auf Basis eines ZSM-5-Zeolithen. Die Reaktionstemperatur beträgt im Allgemeinen 430 bis 500°C, bevorzugt 460 bis 480°C. Der Druck beträgt im Allgemeinen 1,3 bis 2,5 bar. Der erhaltene Rohproduktgasstrom B2 ist vorzugsweise wie folgt zusammengesetzt: 1 bis 15 Gew.-% Ethylen, 1 bis 15 Gew.-% Propylen, 35 bis 80 Gew.-% Wasser, 15 bis 50 Gew.-% $C_2$-$C_6$-Kohlenwasserstoffe, insbesondere Butene und gesättigte $C_4$- und $C_5$-Kohlenwasserstoffe sowie $C_6^+$-Kohlenwasserstoffe und 0,01 bis 1,5 Gew.-% Methanol und Dimethylether.

Der Olefin-Festbettreaktor ist im Allgemeinen als Hordenreaktor ausgestaltet. Die Zahl der Horden beträgt vorzugsweise 4 bis 6. In einer Ausführungsform werden insgesamt bis zu 50 Gew.-% des Gasstroms A direkt einer oder mehreren Horden des Olefin-Festbettreaktors, vorzugsweise allen Horden des Olefin-Festbettreaktors, zugeführt. In einer weiteren Ausführungsform wird Methanol direkt einer oder mehreren Horden des Olefin-Festbettreaktors, vorzugsweise allen Horden des Olefin-Festbettreaktors, zugeführt.

Der Rohproduktgasstrom B weist bei Austritt aus dem Reaktor eine Temperatur von im Allgemeinen 430 bis 520°C, bevorzugt 460 bis 480°C auf.

In einem bevorzugten Schritt C1) wird der Rohproduktgasstrom B in einem oder mehreren Wärmetauschern auf eine Temperatur im Bereich von 170 bis 220°C durch Wärmetausch mit dem Einspeisungsgasstrom A2 abgekühlt. Nach diesem Abkühlschritt beträgt die Temperatur des Rohproduktgasstroms B im Allgemeinen 160 bis 220°C, vorzugsweise 170 bis 210°C, beispielsweise 190°C.

In einem bevorzugten Schritt C2) erfolgt eine weitere Abkühlung des Rohproduktgasstroms B auf eine Temperatur im Bereich von 30 bis 60°C durch Inkontaktbringen mit einem oder mehreren wasserhaltigen Quench-Kreislaufströmen, wobei Wasser und Methanol auskondensiert werden und ein an Wasser und Methanol abgereicherter Kohlenwasserstoff-Rohproduktgasstrom C erhalten wird. Der so erhaltene Kohlenwasserstoff-Rohproduktgasstrom C enthält im Wesentlichen Ethylen, Propylen, weitere $C_2$-$C_6$-Kohlenwasserstoffen und $C_7^+$-Kohlenwasserstoffe.

In einem Schritt D) werden aus dem Kohlenwasserstoff-Rohproduktgasstrom C ein oder mehrere $C_2$-$C_4$-Olefine enthaltende Produktströme abgetrennt und der mindestens eine $C_2$-$C_6$-Kohlenwasserstoffe enthaltende Rückführstrom R gewonnen. Der (Gesamt)rückführstrom R kann mehreren einzelne Rückführströmen R1, R2, R3 usw. umfassen oder aus diesen gebildet werden. Im Allgemeinen enthält der gesamte, $C_2$-$C_6$-Kohlenwasserstoffe enthaltende Rückführstrom R im Wesentlichen, das heißt zu >95 Gew.-%, $C_2$-$C_6$-Kohlenwasserstoffe.

Im Allgemeinen umfasst der Schritt D) die Schritte D1) bis D7):

D1) Kompression des Kohlenwasserstoff-Rohproduktgasstroms C, wobei ein Propylen, $C_4$-, $C_5$- und $C_6^+$-Kohlenwasserstoffe enthaltender flüssiger Kohlenwasserstoffstrom D11 und ein Ethan, Ethen und Propylen enthaltender gasförmiger Kohlenwasserstoffstrom D12 gewonnen werden;

D2) Abtrennung von Wasser aus dem flüssigen Kohlenwasserstoffstrom D11 durch Phasentrennung, wodurch ein flüssiger Kohlenwasserstoffstrom D21 erhalten wird;

D3) Abtrennung eines Propylen enthaltenden Stroms D31 aus dem flüssigen Kohlenwasserstoffstrom D21, wobei ein $C_4$-, $C_5$- und $C_6^+$-Kohlenwasserstoffe enthaltender Strom D32 erhalten wird; oder Abtrennung eines Propylen und $C_4$-Kohlenwasserstoffe enthaltenden Stroms D31, wobei ein $C_4$-, $C_5$- und $C_6^+$-Kohlenwasserstoffe enthaltender Strom D32 erhalten wird;

D4) Abtrennung eines $C_6^+$-Kohlenwasserstoffe enthaltenden Nebenproduktstroms D41 aus dem $C_4$-, $C_5$- und $C_6^+$-Kohlenwasserstoffe enthaltenden Strom D32, wobei ein $C_4$-, $C_5$- und $C_6$-Kohlenwasserstoffe enthaltender Strom D42 erhalten wird; gegebenenfalls enthält der Strom D41 aromatische $C_6$-Kohlenwasserstoffe und der Strom D42 aliphatische $C_6$-Kohlenwassertsoffe;

D5) Abtrennung eines Propylen enthaltenden Stroms D51 aus dem Ethan, Ethen und Propylen enthaltenden gasförmiger Kohlenwasserstoffstrom D12, wobei ein Ethan und Ethen enthaltender Strom D52 erhalten wird;

D6) Abtrennung eines Butene enthaltenden Stroms D61 aus dem $C_4$-, $C_5$- und $C_6$-Kohlenwasserstoffe enthaltende Strom D42, wobei ein $C_5$- und $C_6$-Kohlenwasserstoffe enthaltender Strom D62 erhalten wird; und/oder Abtrennung eines Propylen enthaltenden Stroms D63 aus dem Strom D31, wobei ein Butene enthaltender Strom D64 erhalten wird;

D7) Gewinnung mindestens eines Rückführstroms R aus einem oder mehreren der Ströme ausgewählt aus dem $C_4$-, $C_5$- und $C_6$-Kohlenwasserstoffe enthaltender Strom D42, dem $C_5$- und $C_6$-Kohlenwasserstoffe enthaltenden Strom D62, dem Propylen enthaltenden Strom D31, dem Propylen enthaltenden Strom D51, dem Propylen enthaltenden Strom D63, dem Butene enthaltenden Strom D61, dem Butene enthaltenden Strom D64 und dem Ethan und Ethen enthaltenden Strom D52.

Die Schritte D3), D4), D5) und D6) werden in üblichen Destillationsvorrichtungen durchgeführt. Als Destillationsvorrichtungen kommen grundsätzlich die dem Fachmann für derartige Trennaufgaben bekannten Vorrichtungen in Frage. Neben dem eigentlichen Kolonnenkörper mit Einbauten enthält die Destillationskolonne wie üblich noch einen Kopfkondensator und einen Sumpfverdampfer. Der Kolonnenkörper kann beispielsweise mit Packungen, Füllkörpern oder Böden bestückt sein. Die Destillationsvorrichtungen können mit dem allgemeinen Wissen des Fachmanns ausgelegt und betrieben werden.

In Schritt E) wird ein Teil der $C_2$-$C_4$-Oefine und mindestens ein Teil der $C_5$-$C_6$-Kohlenwasserstoffe als ein oder mehrere Kohlenwasserstoff-Rückführströme R in Schritt B) zurückgeführt.

In Schritt F) wird ein Propylen enthaltender Wertproduktstrom, ein Ethylen enthaltender Wertproduktstrom und gegebenenfalls ein Butene enthaltender Wertproduktstrom gewonnen.

Propylen kann als Wertprodukt aus den Strömen D31 oder D63 und D51 gewonnen werden. Ethylen kann als Wertprodukt aus dem Strom D52 gewonnen werden. Butene können aus dem Strom D61 und/oder D64 gewonnen werden.

Der oder die Rückführströme R können aus einem oder mehreren der oben beschriebenen Ströme D31, D42, D51, D52, D61, D62, D63 und D64 gewonnen werden.

Bezogen auf 100 Gew.-% der als Wertprodukte gewonnenen $C_2$-$C_4$-Olefine werden 30 bis 60 Gew.-% Ethylen, 30 bis 60 Gew.-% Propylen und 0 bis 30 Gew.-% Butene als Wertprodukte gewonnen und, bezogen auf die in dem Rohproduktstrom B enthaltenen $C_2$-$C_4$-Oefine, 0 bis 40% des Ethylens, 40 bis 90% des Propylens und 0 bis 100% der Butene in Schritt B) zurückgeführt, oder es werden, bezogen auf 100 Gew.-% der als Wertprodukte gewonnenen $C_2$-$C_4$-Olefine, 0 bis 20 Gew.-% Ethylen, 70 bis 100 Gew.-% Propylen und 1 bis 20 Gew.-% Butene als Wertprodukte gewonnen, und, bezogen auf die in dem Rohproduktstrom B enthaltenen $C_2$-$C_4$-Oefine, 0 bis 100 % des Ethylens, 0 bis 20 % des Propylens und 40 bis 100% der Butene in Schritt B) zurückgeführt.

In Schritt G) wird ein $C_6^+$-Kohlenwasserstoffe enthaltenden Nebenproduktstrom aus dem Verfahren ausgeschleust. Dabei kann es sich um den Strom D41 handeln.

Die Erfindung wird durch die nachstehenden Beispiele näher erläutert.

Beispiele

Es wurden die nachstehenden Bereiche 1 - 4 der Zusammensetzung von Einspeisungsstrom (Ethanol-Gehalt), Rohproduktstrom (Verhältnisse $C_2$/$C_3$-Olefine und $C_4$/$C_3$-Olefine) und gewonnenem Wertprodukt (Anteile $C_2$-, $C_3$- und $C_4$-Olefine) rechnerisch simuliert. Angabe in Gewichtsverhältnissen.

Zur Ermittlung der Vorzugsbereiche wurden im Labormaßstab katalytische Experimente durchgeführt und auf deren Basis die zu erwartenden Umsätze und Massenströme in einem industriellen Prozess mit Vorreaktor, Hordenreaktoren und Auftrennsektion berechnet. Eine Optimierung dieses Systems führte zu den in Bereichen 1 - 4, die in Tabelle 1 angegeben sind. Tabelle 2 zeigt die relevanten Strommengen für ermittelte Beispielpunkte in Bereichen 1 und 2. Ähnliche Beispielwerte für Bereich 3 und 4 sind in Tabelle 3 aufgeführt. Durch die Zugabe von Ethanol kann jeweils der relative Rückführstrom (Recycle) sowie der relative Nebenproduktstrom minimiert und so die Energie- und Masseneffizienz des Prozesses verbessert werden.

Tabelle 1

| | Verhältnis C$_2$/C$_3$-Olefine im Wertprodukt | Verhältnis C$_4$/C$_3$-Olefine im Wertprodukt | Anteil rückgeführtes am gesamten C2-Olefin | Anteil rückgeführtes am gesamten C3-Olefin | Anteil rückgeführtes am gesamten C4-Olefin | $\dfrac{EtOH}{EtOH + MeOH}$ |
|---|---|---|---|---|---|---|
| Bereich 1 | 0,8-1,2 | 0-0,7 | 0-0,4 | 0,4-0,9 | 0-1 | |
| Bereich 2 | 0,8-1,2 | 0-0,7 | 0-0,4 | 0,4-0,9 | 0-1 | 0,3-0,5 |
| Bereich 3 | 0-0,2 | 0,01-0,2 | 0-1 | 1 | 0-0,6 | |
| Bereich 4 | 0-0,2 | 0,01-0,2 | 0-1 | 1 | 0-0,6 | 0,01-0,3 |

Die Ergebnisse der Simulationen sind in Tabelle 2 und 3 zusammengefasst. Alle Angaben in Gewichtsverhältnissen.

Tabelle 2

| $\dfrac{EtOH}{EtOH + MeOH}$ | 0 | | | 0,5 | | |
|---|---|---|---|---|---|---|
| | Produkt | Recycle | Nebenprodukte | Produkt | Recycle | Nebenprodukte |
| Rel. Massenstrom | 1,0 | 3,73 | 1,88 | 1,0 | 0,63 | 0,53 |
| MeOH | | | | | | |
| EtOH | | | | | | |
| DME | | 0,01 | 0,01 | | | 0,02 |
| H2O | | | | | | |
| H2 | | | | | | |
| CO2 | | | | | | |
| CH4 | 0,02 | 0,01 | | | | |
| C2H40,390,41 | | | | | | |
| C3H60,410,390,410,66 | | | | | | |
| C4H80,200,120,180,03 | | | | | | |
| C5H100,010,020,020,02 | | | | | | |
| C6-C8-Olefine | | 0,01 | 0,01 | | 0,02 | 0,03 |
| C2H6 | | | 0,08 | | | 0,04 |
| C3H8 | | 0,20 | 0,11 | | 0,11 | 0,13 |
| C4H10 | | 0,18 | 0,16 | | 0,02 | 0,19 |
| C5H12 | | 0,05 | 0,11 | | 0,07 | 0,08 |
| C6-C8 Paraffine | | 0,03 | 0,06 | | 0,06 | 0,08 |
| Aromaten | | | 0,42 | | | 0,39 |

Tabelle 3

| $\dfrac{EtOH}{EtOH + MeOH}$ | 0 | | | 0,21 | | |
|---|---|---|---|---|---|---|
| | Produkt | Recycle | Nebenprodukte | Produkt | Recycle | Nebenprodukte |
| Rel. Massenstrom | 1,0 | 1,09 | 0,65 | 1,0 | 1,02 | 0,56 |
| MeOH | | | | | | |
| EtOH | | | | | | |
| DME | | 0,02 | 0,02 | | 0,01 | 0,02 |
| H2O | | | | | | |
| H2 | | | | | | |
| CO2 | | | | | | |
| CH4 | | | 0,01 | | | 0,01 |
| C2H4 | 0,02 | 0,15 | | 0,03 | 0,23 | |

(fortgesetzt)

| | Produkt | Recycle | Nebenprodukte | Produkt | Recycle | Nebenprodukte |
|---|---|---|---|---|---|---|
| C3H6 | 0,85 | | | 0,86 | | |
| C4H8 | 0,13 | 0,18 | | 0,11 | 0,16 | |
| C5H10 | | 0,01 | 0,02 | | 0,01 | 0,02 |
| C6-C8-Olefine | | 0,01 | 0,02 | | 0,01 | 0,03 |
| C2H6 | | 0,36 | 0,07 | | 0,34 | 0,07 |
| C3H8 | | | 0,10 | | | 0,12 |
| C4H10 | | 0,16 | 0,18 | | 0,14 | 0,17 |
| C5H12 | | 0,07 | 0,12 | | 0,06 | 0,10 |
| C6-C8 Paraffine | | 0,04 | 0,06 | | 0,04 | 0,07 |
| Aromaten | | | 0,39 | | | 0,39 |

**Patentansprüche**

1. Verfahren zur Herstellung von $C_2$-$C_4$-Olefinen aus Methanol und Ethanol mit den Schritten:

A) Einspeisen eines Methanol und gegebenenfalls Ethanol enthaltenden Einspeisungsstroms A in einen Dimethylether-Festbettreaktor und katalytische Umsetzung von Methanol zu Dimethylether, wobei ein Produktstrom A1 enthaltend Dimethylether, Methanol, Ethanol und Wasserdampf erhalten wird;
B) Mischen des Stroms A1 mit mindestens einem Kohlenwasserstoff-Rückführstrom R enthaltend $C_2$-$C_6$-Kohlenwasserstoffe und katalytische Umsetzung in einem Olefin-Festbettreaktor zu einem Rohproduktstrom B enthaltend $C_2$-$C_4$-Olefine, $C_5$-$C_6$-Kohlenwasserstoff und $C_7^+$-Kohlenwasserstoffe;
C) Abkühlung des Rohproduktstroms B, wobei ein Kohlenwasserstoff-Rohproduktstrom C erhalten wird;
D) Auftrennung des Kohlenwasserstoff-Rohproduktstroms C in einen Propylen enthaltenden Wertproduktstrom, gegebenenfalls einen Ethylen enthaltenden Wertproduktstrom, einen Butene enthaltenden Wertproduktstrom, mindestens einen $C_5$-$C_6$-Kohlenwasserstoff enthaltenden Rückführstrom und mindestens einen $C_6^+$-Kohlenwasserstoffe enthaltenden Nebenproduktstrom;
E) Rückführung eines Teils der $C_2$-$C_4$-Oefine und mindestens eines Teils der $C_5$-$C_6$-Kohlenwasserstoffe als ein oder mehrere Kohlenwasserstoff-Rückführströme in Schritt B);
F) Gewinnung eines Propylen enthaltenden Wertproduktstroms, eines Ethylen enthaltenden Wertproduktstroms und gegebenenfalls eines Butene enthaltenen Wertproduktstroms;
G) Ausschleusung des $C_6^+$-Kohlenwasserstoffe enthaltenden Nebenproduktstroms; **dadurch gekennzeichnet, dass**

der Strom A, bezogen auf Methanol und Ethanol, < 1 Gew.-% oder 30 bis 50 Gew.-% Ethanol enthält, wobei, bezogen auf 100 Gew.-% der als Wertprodukte gewonnenen C2-C4-Olefine, 30 bis 60 Gew.-% Ethylen, 30 bis 60 Gew.-% Propylen und 0 bis 30 Gew.-% Butene als Wertprodukte gewonnen werden, und, bezogen auf die in dem Rohproduktstrom B enthaltenen $C_2$-$C_4$-Olefine, 0 bis 40% des Ethylens, 40 bis 90% des Propylens und 0 bis 100% der Butene in Schritt B) zurückgeführt werden,
oder der Strom A, bezogen auf Methanol und Ethanol, 1 bis 30 Gew.-% Ethanol enthält, wobei, bezogen auf 100 Gew.-% der als Wertprodukte gewonnenen $C_2$-$C_4$-Olefine, 0 bis 20 Gew.-% Ethylen, 70 bis 100 Gew.-% Propylen und 1 bis 20 Gew.-% Butene als Wertprodukte gewonnen werden, und, bezogen auf die in dem Rohproduktstrom B enthaltenen C2-C4-Oefine, 0 bis 100 % des Ethylens, 0 bis 20 % des Propylens und 40 bis 100% der Butene in Schritt B) zurückgeführt werden.

2. Verfahren nach Anspruch 1, wobei die Schritte A) bis D) die nachfolgenden Schritte A1), A2), B1), B2), C1), C2) und D) umfassen:

A1) Einspeisen eines Methanol und Ethanol enthaltenden Einspeisungsstroms A in einen Dimethylether-Festbettreaktor und katalytische Umsetzung von Methanol zu Dimethylether, wobei ein Produktstrom A1 enthaltend Dimethylether, Methanol, Ethanol und Wasserdampf erhalten wird;

A2) Mischen von mindestens einem Teil des Produktstroms A1 mit mindestens einem Kohlenwasserstoff-Rückführstrom R enthaltend $C_2$-$C_6$-Kohlenwasserstoffe und einem Wasserdampfstrom, wobei ein zweiter Einspeisungsstrom A2 erhalten wird;

B1) Aufheizen des zweiten Einspeisungsstroms A2 in einem oder mehreren Wärmetauschern auf eine Temperatur im Bereich von 430 bis 500°C und Einspeisung in einen Olefin-Festbettreaktor, wobei das Aufheizen auch vor dem Mischen einzelner Teilströme zum Einspeisungsstrom A2 in Schritt A2) stattfinden kann;

B2) katalytische Umsetzung des Einspeisungsstroms A2 bei einer Temperatur im Bereich von 430 bis 520°C zu einem Rohproduktgasstrom B enthaltend Ethylen, Propylen, Butene, weitere $C_2$-$C_6$-Kohlenwasserstoffe, $C_7^+$-Kohlenwasserstoffe, Methanol und Wasserdampf;

C1) Abkühlen des Rohproduktgasstroms B in einem oder mehreren Wärmetauschern auf eine Temperatur im Bereich von 170 bis 220°C durch Wärmetausch mit dem Einspeisungsstrom A2;

C2) weitere Abkühlung des Rohproduktgasstroms B auf eine Temperatur im Bereich von 30 bis 60°C durch Inkontaktbringen mit mindestens einem wasserhaltigem Quench-Kreislaufstrom K, wobei Wasser und Methanol auskondensiert werden und ein an Wasser und Methanol abgereicherter Kohlenwasserstoff-Rohproduktgasstrom C2 erhalten wird;

D) Auftrennung des Kohlenwasserstoff-Rohproduktgasstrom C in einen Propylen enthaltenden Wertproduktstrom, gegebenenfalls einen Ethylen enthaltenden Wertproduktstrom, einen Butene enthaltenden Wertproduktstrom, mindestens einen $C_5$-$C_6$-Kohlenwasserstoffe enthaltenden Rückführstrom und mindestens einen $C_6^+$-Kohlenwasserstoffe enthaltenden Nebenproduktstrom.

3. Verfahren nach Anspruch 1 oder 2, **dadurch gekennzeichnet, dass** Schritt D) die Schritte D1) bis D7) umfasst:

D1) Kompression des Kohlenwasserstoff-Rohproduktgasstroms C, wobei ein Propylen, $C_4$-, $C_5$- und $C_6^+$-Kohlenwasserstoffe enthaltender flüssiger Kohlenwasserstoffstrom D11 und ein Ethan, Ethen und Propylen enthaltender gasförmiger Kohlenwasserstoffstrom D12 gewonnen werden;

D2) Abtrennung von Wasser aus dem flüssigen Kohlenwasserstoffstrom D11 durch Phasentrennung, wodurch ein flüssiger Kohlenwasserstoffstrom D21 erhalten wird;

D3) Abtrennung eines Propylen enthaltenden Stroms D31 aus dem flüssigen Kohlenwasserstoffstrom D21, wobei ein $C_4$-, $C_5$- und $C_6^+$-Kohlenwasserstoffe enthaltender Strom D32 erhalten wird; oder Abtrennung eines Propylen und $C_4$-Kohlenwasserstoffe enthaltenden Stroms D31, wobei ein $C_4$-, $C_5$- und $C_6^+$-Kohlenwasserstoffe enthaltender Strom D32 erhalten wird;

D4) Abtrennung eines $C_6^+$-Kohlenwasserstoffe enthaltenden Nebenproduktstroms D41 aus dem $C_4$-, $C_5$- und $C_6^+$-Kohlenwasserstoffe enthaltenden Strom D32, wobei ein $C_4$-, $C_5$- und $C_6$-Kohlenwasserstoffe enthaltender Strom D42 erhalten wird; gegebenenfalls enthält der Strom D41 aromatische $C_6$-Kohlenwasserstoffe und der Strom D42 aliphatische $C_6$-Kohlenwassertsoffe;

D5) Abtrennung eines Propylen enthaltenden Stroms D51 aus dem Ethan, Ethen und Propylen enthaltenden gasförmiger Kohlenwasserstoffstrom D12, wobei ein Ethan und Ethen enthaltender Strom D52 erhalten wird;

D6) Abtrennung eines Butene enthaltenden Stroms D61 aus dem $C_4$-, $C_5$- und $C_6$-Kohlenwasserstoffe enthaltende Strom D42, wobei ein $C_5$- und $C_6$-Kohlenwasserstoffe enthaltender Strom D62 erhalten wird; und/oder Abtrennung eines Propylen enthaltenden Stroms D63 aus dem Strom D31, wobei ein Butene enthaltender Strom D64 erhalten wird;

D7) Gewinnung mindestens eines Rückführstroms R aus einem oder mehreren der Ströme ausgewählt aus dem $C_4$-, $C_5$- und $C_6$-Kohlenwasserstoffe enthaltender Strom D42, dem $C_5$- und $C_6$-Kohlenwasserstoffe enthaltenden Strom D62, dem Propylen enthaltenden Strom D31, dem Propylen enthaltenden Strom D51 dem Propylen enthaltenden Strom D63, dem Butene enthaltenden Strom D61, dem Butene enthaltenden Strom D64 und dem Ethan und Ethen enthaltenden Strom D52.

Europäisches Patentamt
European Patent Office
Office européen des brevets

**EUROPÄISCHER RECHERCHENBERICHT**

Nummer der Anmeldung

EP 22 19 5156

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A,D | US 2010/145125 A1 (XIE ZAIKU [CN] ET AL) 10. Juni 2010 (2010-06-10)<br>* Beispiele 1-24 *<br>* Ansprüche 1-11 *<br>* Absatz [0027] – Absatz [0028] *<br>----- | 1-3 | INV.<br>C07C1/20<br>C07C11/04<br>C07C11/06<br>C07C11/08<br>C07C41/09 |
| A,D | CN 216 106 699 U (UNIV SHANGHAI TRAFFIC) 22. März 2022 (2022-03-22)<br>* Beispiele 1-3 *<br>* Ansprüche 1-10 *<br>----- | 1-3 | C07C43/04<br>C07C4/06 |

RECHERCHIERTE SACHGEBIETE (IPC)

C07C

Der vorliegende Recherchenbericht wurde für alle Patentansprüche erstellt

| Recherchenort | Abschlußdatum der Recherche | Prüfer |
|---|---|---|
| Den Haag | 3. Februar 2023 | Delanghe, Patrick |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

............................................................................
& : Mitglied der gleichen Patentfamilie, übereinstimmendes Dokument

EPO FORM 1503 03.82 (P04C03)

**EP 4 335 836 A1**

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

**03-02-2023**

| Im Recherchenbericht angeführtes Patentdokument | Datum der Veröffentlichung | Mitglied(er) der Patentfamilie | Datum der Veröffentlichung |
|---|---|---|---|
| US 2010145125 A1 | 10-06-2010 | AU 2008213545 A1 | 14-08-2008 |
| | | BR PI0806359 A2 | 06-09-2011 |
| | | CN 101239870 A | 13-08-2008 |
| | | US 2010145125 A1 | 10-06-2010 |
| | | WO 2008095417 A1 | 14-08-2008 |
| CN 216106699 U | 22-03-2022 | KEINE | |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2010145125 A1
- CN 216106699 U
- CN 110218138 A
- WO 2017102096 A1